(19) Europäisches Patentamt
European Patent Office
Office européen des brevets

(11) **EP 2 043 539 B1**

(12) **EUROPÄISCHE PATENTSCHRIFT**

(45) Veröffentlichungstag und Bekanntmachung des Hinweises auf die Patenterteilung:
**26.09.2012   Patentblatt 2012/39**

(21) Anmeldenummer: **07786043.5**

(22) Anmeldetag: **12.07.2007**

(51) Int Cl.:
***A61B 18/12*** *(2006.01)*

(86) Internationale Anmeldenummer:
**PCT/EP2007/006215**

(87) Internationale Veröffentlichungsnummer:
**WO 2008/009385 (24.01.2008 Gazette 2008/04)**

(54) **ELEKTRODENEINRICHTUNG**

ELECTRODE DEVICE

SYSTEME D'ELECTRODES

(84) Benannte Vertragsstaaten:
**DE FR GB IT**

(30) Priorität: **19.07.2006   DE 102006033510**
**02.05.2007   DE 102007020583**

(43) Veröffentlichungstag der Anmeldung:
**08.04.2009   Patentblatt 2009/15**

(73) Patentinhaber: **ERBE Elektromedizin GmbH**
**72072 Tübingen (DE)**

(72) Erfinder: **SELIG, Peter**
**72379 Hechingen (DE)**

(74) Vertreter: **Bohnenberger, Johannes**
**Meissner, Bolte & Partner GbR**
**Widenmayerstrasse 48**
**80538 München (DE)**

(56) Entgegenhaltungen:
**EP-A- 0 262 888       WO-A-2006/041756**
**CA-A1- 1 219 642      DE-A1- 3 206 947**
**DE-A1- 3 239 640**

EP 2 043 539 B1

**Beschreibung**

Die Erfindung betrifft eine Elektrodeneinrichtung, insbesondere eine Neutralelektrode zum Einleiten eines hochfrequenten Behandlungsstroms nach dem Oberbegriff des Patentanspruches 1.

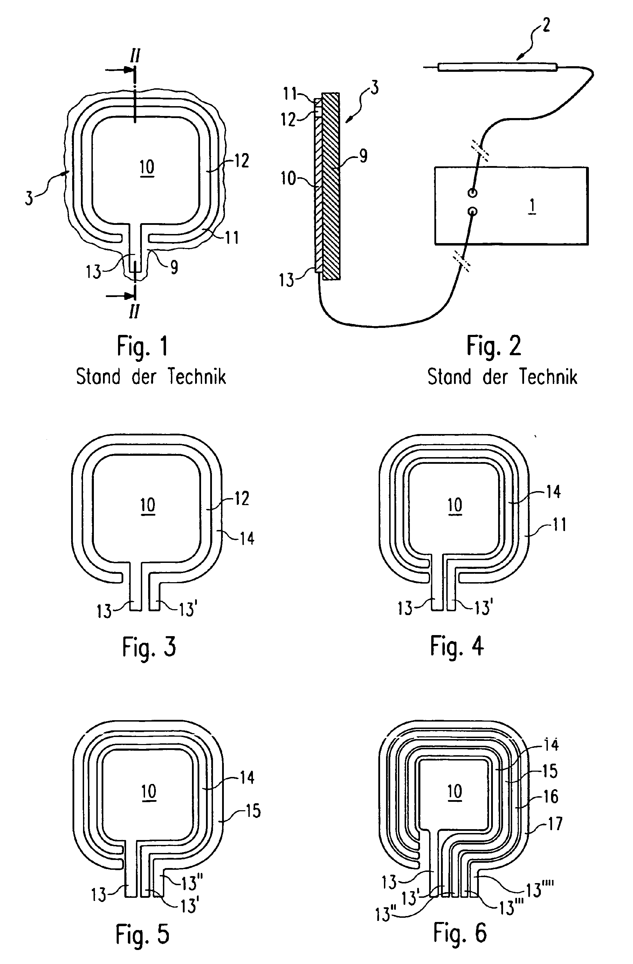

Bei elektrochirurgischen Eingriffen werden unter anderem so genannte monopolare Instrumente verwendet, bei welchen der in das Gewebe eingeleitete Behandlungsstrom durch den Körper in eine so genannte Neutralelektrode fließt. Eine derartige Anordnung ist im Prinzip in den Fig. 1 und 2 gezeigt. Sie umfasst einen Generator 1, der einen hochfrequenten elektrischen Strom liefert (üblich sind 350 kHz), an den einerseits ein elektrochirurgisches Instrument 2 und andererseits eine Neutralelektrode 3 angeschlossen ist. Die Neutralelektrode 3 wird unter Zwischenlage eines leitfähigen Gels auf eine Hautschicht eines zu behandelnden Patienten aufgeklebt.

Aus der EP 1 173 095 B1 ist eine Neutralelektrode bekannt, die einen Aufbau entsprechend dem nach den Fig. 1 und 2 aufweist. Die Neutralelektrode 3 weist eine Trägerfolie 9 auf, auf welcher eine Hauptelektrode 10 angebracht ist, die über eine Anschlussfahne 13 mit dem Generator 1 verbunden werden kann. Rings um die Hauptelektrode 10 ist ein elektrisch leitender Ring 11 vorgesehen, der von der Hauptelektrode 10 durch einen Spalt 12 getrennt ist. Ein solcher elektrisch leitender Ring führt zu einer verbesserten Stromaufteilung auf dem kontaktierten Hautabschnitt bzw. dem darunter liegenden Gewebe. Hierbei ist zu bedenken, dass die verschiedenen Gewebearten sehr unterschiedliche spezifische Widerstände aufweisen. Während Muskeln einen spezifischen Widerstand von 0,2 mΩcm (bei 1 MHz) aufweisen, liegt der spezifische Widerstand von Blut bei 0,16 mΩcm und von Fett bei 3,3 mΩcm. Weiterhin können sich die aufgeklebten Neutralelektroden insbesondere vom Rand her leicht ablösen, so dass stellenweise der Kontakt unterbrochen wird.

Zur Feststellung eines korrekten Elektrodensitzes sind nun viele Vorschläge gemacht worden. So z.B. wird in der EP 0 390 937 B1 eine Einrichtung zur Überwachung der Applikation von Neutralelektroden bei der Hochfrequenzchirurgie vorgeschlagen, wobei mit zwei gleichgroßen Elektroden gearbeitet wird und ein Differenzwiderstand zwischen den beiden Elektroden gemessen wird. Aus dem Messergebnis wird auf einen korrekten oder fehlerhaften Sitz der Elektrode rückgeschlossen.

Aus der EP 1 051 949 B1 ist eine Neutralelektrode mit einer Impedanz-Messeinrichtung der eingangs genannten Art bekannt, wobei allerdings exaktere Anweisungen zum Aufbau der Elektrode aus dieser Druckschrift nicht hervorgehen.

In allen Fällen ist der bisherige Elektrodenaufbau bzw. ist das bisherige Verfahren nur bedingt geeignet, mit vertretbarem Aufwand ein korrektes Einleiten eines hochfrequenten Behandlungsstromes über eine Neutralelektrode sicherzustellen.

Der Erfindung liegt die Aufgabe zu Grunde, eine Elektrodeneinrichtung und ein Verfahren zur Überwachung der Anlage und des HF-Stromflusses und seiner räumlichen Verteilung der eingangs genannten Art dahin gehend aufzuzeigen, dass eine erhöhte Sicherheit bei verbesserten Stromeinleitungseigenschaften sichergestellt wird.

Diese Aufgabe wird vorrichtungsmäßig durch eine Elektrodeneinrichtung nach Anspruch gelöst.

Dokument DE-A-3239640 offenbart eine Vorrichtung gemäß Oberbegriff des Anspruchs 1.

Der Messstrom weigt eine vom Behandlungsstrom derart verschiedene Frequenz oder Phasenlage auf, dass der Messstrom vom Behandlungsstrom durch Filtereinrichtungen trennbar ist. Der Messstrom ist hierbei auch ein hochfrequenter Strom, wie dies die Vorschriften betreffend elektrochirurgische Geräte fordern. Da sowohl der Messstrom als auch der Behandlungsstrom durch die entsprechenden Schaltungsteile definiert sind, können sehr schmalbandige Filter (z.B. mittels einer PLL-Schaltung) aufgebaut werden, so dass sehr genaue Messungen zu erwarten sind.

Vorzugsweise ist die Frequenz des Messstromes niedriger als die Frequenz des Behandlungsstromes. Damit kommt der Messstrom nicht in den Frequenzbereich von Oberwellen, die z.B. durch die Entstehung von Lichtbögen bei der Elektrochirurgie erzeugt worden.

Die Impedanz-Messeinrichtung weist eine Spannungsmesseinrichtung zur Messung eines Spannungsabfalls zwischen der Hauptelektrode und der Messelektrode beim Durchleiten des Messstroms auf. Es muss also der Generator zur Erzeugung des Messstromes lediglich als Wechselstromquelle mit konstanter Amplitude ausgebildet werden, was schaltungstechnisch sehr einfach zu realisieren ist.

Weiterhin weist die Impedanz-Messeinrichtung eine Spannungsmesseinrichtung zur Messung eines Spannungsabfalls zwischen der Hauptelektrode und der Messelektrode beim Fließen eines Behandlungsstromes auf. Dadurch können in einfacher Weise Messwerte generiert werden, die eine Aussagekraft in Bezug auf die zu erwartende Erwärmung des Gewebes bzw. des Hautabschnittes haben.

Die Messelektrode (bzw. die mehreren Messelektroden) wird vorzugsweise als elektrisch leitender Ring ausgebildet, übernimmt also gleichzeitig die oben bereits beschriebene und aus dem Stand der Technik nach der EP 1 173 095 B1 bekannte Funktion einer Verringerung der Stromdichte und darum Verminderung der Erwärmung des Gewebes.

Bei einer bevorzugten Ausführungsform ist eine Vielzahl von insbesondere jeweils elektrisch leitenden Ringen bildenden Messelektroden und eine Vielzahl von Impedanz-Messeinrichtungen vorgesehen. Die Auswerteinrichtung ist derart ausgebildet, dass ein Verlauf der Spannungsabfälle des Behandlungsstroms von der Hauptelektrode zu den Messelektroden feststellbar ist. Mit einer derartigen Anordnung ist es möglich, eine Inhomogenität des Gewebes hinsichtlich seines spezifischen Widerstands unter der Elektrodeneinrichtung festzustellen. Darübet hinaus ist ein Ablösen der Elektrodeneinrichtung mit einer derartigen Anordnung besonders leicht feststellbar.

Die Auswerteinrichtung ist vorzugsweise derart ausgebildet, dass ein Warnsignal dann abgegeben wird, wenn ein durch den Behandlungsstrom bewirkter Spannungsabfall zur Messelektrode eine vorbestimmte Schwelle, abhängig von $R_{UEB}$, überschreitet. Dadurch wird die Sicherheit der Elektrodeneinrichtung weiter erhöht.

Nachfolgend werden bevorzugte Ausführungsformen der Erfindung anhand von Abbildungen näher erläutert. Hierbei zeigen

- Fig. 1 eine Draufsicht auf eine bekannte Elektrodeneinrichtung,

- Fig. 2 einen Schnitt durch die Elektrodeneinrichtung nach Fig. 1 entlang der Linie II-II,

- Fig. 3 eine Draufsicht auf eine erste bevorzugte Ausführungsform der Erfindung in einer Ansicht entsprechend der nach Fig. 1,

- Fig. 4 eine zweite Ausführungsform der Erfindung in einer Ansicht entsprechend der nach Fig. 3,

- Fig. 5 eine dritte Ausführungsform der Erfindung in einer Ansicht entsprechend der nach Fig. 3,

- Fig. 6 eine vierte Ausführungsform der Erfindung in einer Ansicht entsprechend der nach Fig. 3,

- Fig. 7 einen Teilschnitt durch eine Hauptelektrode und das darunter liegende Gewebe in einer symbolischen Darstellung,

- Fig. 8 eine Darstellung entsprechend der nach Fig. 7, jedoch mit einer zusätzlichen Equipotentialelektrode,

- Fig. 9 eine Darstellung entsprechend der nach Fig. 8 mit zusätzlich eingezeichneten Widerstandsverhältnissen,

- Fig. 10 einen Schnitt durch eine Ausführungsform der Erfindung gemäß Fig. 3 mit dazu eingezeichneten peripheren Bauteilen,

- Fig. 11 eine Darstellung entsprechend der nach Fig. 10, jedoch bei anderen Verhältnissen im Gewebe und

- Fig. 12 eine Darstellung entsprechend der nach Fig. 10, mit einem zusätzlichen elektrisch leitenden Ring.

In der nachfolgenden Beschreibung werden für gleiche und gleich wirkende Teile dieselben Bezugsziffern verwendet.

Bei der in Fig. 3 gezeigten Ausführungsform der Erfindung ist - wie beim Stand der Technik - eine Hauptelektrode 10 im Zentrum der Elektrodenanordnung vorgesehen. Durch einen Spalt 12 getrennt von der Hauptelektrode 10 ist ein elektrisch leitender Ring 11 vorgesehen, der einerseits die Hauptelektrode 10 umgibt und der andererseits sich mit einem Finger (immer noch unter Trennung durch einen Spalt 12) in die Hauptelektrode 10 hinein erstreckt. Die Hauptelektrode 10 ist mit einer Anschlussfahne 13 versehen, während der elektrisch leitende Ring, der in diesem Fall eine Messelektrode 14 bildet, über eine Anschlussfahne 13' wie weiter unten beschrieben mit der elektrischen Schaltung verbunden ist.

Die Ausführungsform nach Fig. 4 unterscheidet sich von der nach Fig. 3 dadurch, dass zusätzlich zur Messelektrode 14 ein elektrisch leitender Ring 11 die Hauptelektrode 10 und die Messelektrode 14 (durch einen Spalt getrennt) umgibt. Der elektrisch leitende Ring 14 weist also keine Anschlussfahne auf. Die Ausführungsform nach Fig. 5 unterscheidet sich von der nach Fig. 4 dadurch, dass der in Fig. 4 vorgesehene elektrisch leitende Ring 11 nunmehr als weitere Messelektrode 15 ausgebildet ist, also eine Anschlussfahne 13" aufweist, über welche ein Anschluss an periphere Bauteile erfolgen kann.

Die Ausführungsform nach Fig. 6 unterscheidet sich von der nach Fig. 5 dadurch, dass insgesamt vier Messelektroden 14, 15, 16 und 17 vorgesehen sind, die allesamt die Hauptelektrode in immer weiteren Abständen jeweils von ihr und voneinander durch Spalte getrennt umgeben.

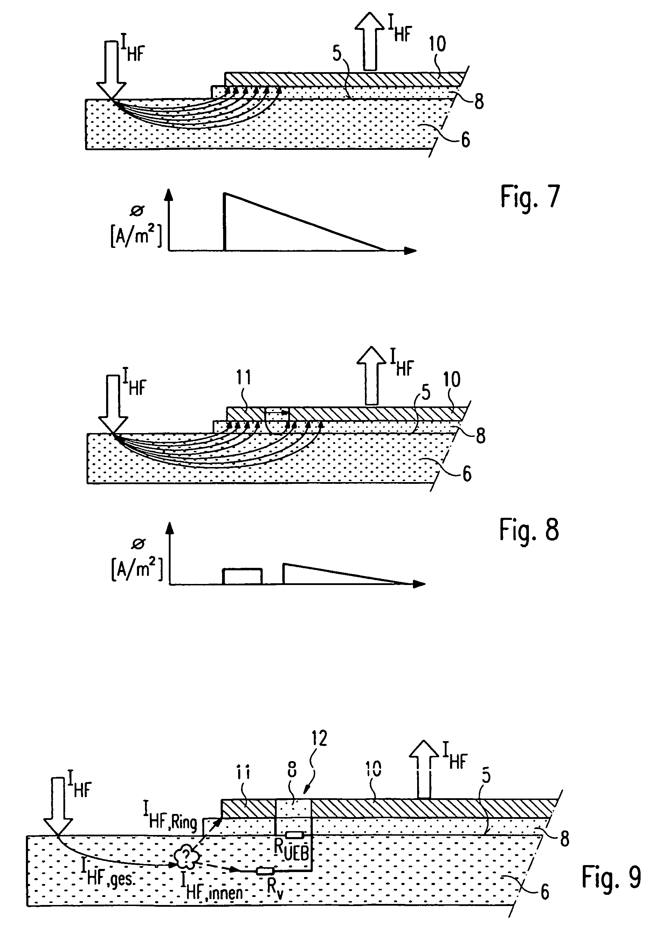

Ein wesentlicher Teilaspekt der hier vorliegenden Problematik wird nachfolgend anhand der Fig. 7 - 9 näher erläutert.

Wenn man von einer "einfachen" Anordnung ausgeht, die also nur eine einzige Hauptelektrode 10 aufweist, welche über ein Leitgel 8 auf einen Hautabschnitt 5 aufgeklebt ist, so fließt ein Behandlungsstrom $I_{HF}$ vom (hier nicht gezeigten) chirurgischen Instrument in das Gewebe 6 des zu behandelnden Patienten ein. Im Gewebe 6 fließt dieser Strom weiter und durch den Hautabschnitt 5 über das Leitgel 8 in die Hauptelektrode 10 hinein und von dort zum entsprechenden Gegenpol des verwendeten (nicht gezeigten) Hochfrequenz-Generators. Der Behandlungsstrom $I_{HF}$ wählt nun immer den kürzesten Weg, so dass die Stromdichte an dem Abschnitt der Hauptelektrode 10 am höchsten ist, welcher dem Behandlungsort (wo der Behandlungsstrom in das Gewebe 6 einfließt) am höchsten ist. Darüber hinaus findet an den Rändern der Hauptelektrode 10 ohnehin immer eine Stromkonzentration statt. Dies ist in Fig. 7 schematisch mit dem Diagramm unter der Schnittzeichnung dargestellt.

Die in Fig. 8 gezeigte Anordnung unterscheidet sich von der nach Fig. 7 durch einen dort vorgesehenen elektrisch leitenden Ring 11. Aufgrund der bestehenden Widerstandsverhältnisse, die in Fig. 9 in vereinfachter Form wiedergegeben sind, wird durch den elektrisch leitenden Ring 11 eine "Entlastung" des Gewebes insofern bewerkstelligt, als ein Teil des Behandlungsstromes in den elektrisch leitenden Ring 11 einfließt und (unter anderem über das Leitgel 8) von dort aus in die Hauptelektrode 10 gelangt. Dadurch ergibt sich eine Verringerung der Stromdichte, wie sie schematisch im Diagramm in Fig. 8 angedeutet ist.

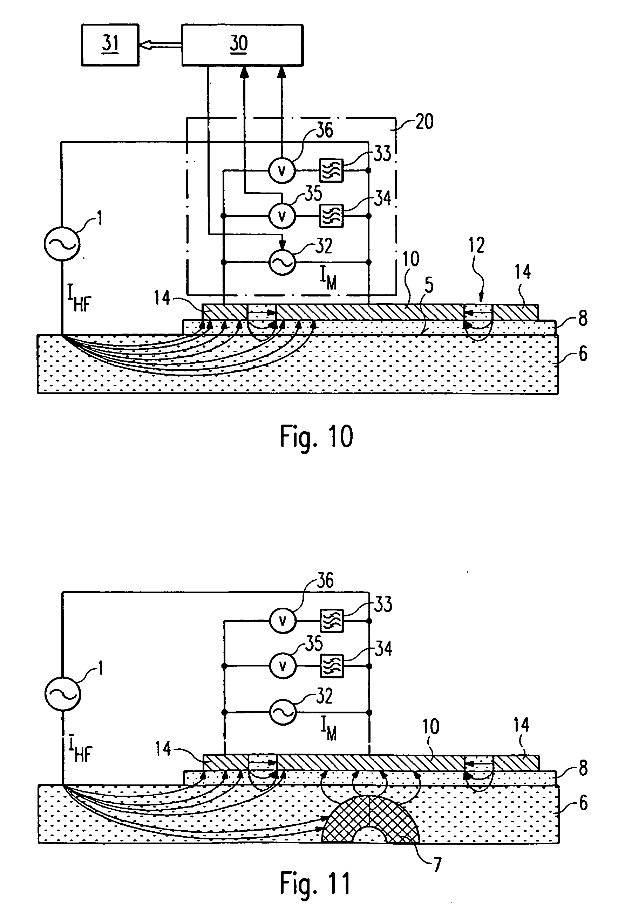

Bei der in Fig. 10 gezeigten Ausführungsform der Erfindung ist nun die Anordnung ähnlich der nach Fig. 9, jedoch bildet der in Fig. 9 vorgesehene elektrisch leitende Ring hier eine erste Messelektrode 14.

Es ist eine Impedanz-Messeinrichtung 20 vorgesehen, in welcher ein Messstromgenerator 32 angeordnet ist, der mit seinen Ausgängen einerseits mit der Hauptelektrode 10 und andererseits mit der Messelektrode 14 verbunden ist. Der Messstromgenerator 32 liefert einen konstanten Messstrom $I_M$, dessen Frequenz niedriger ist als die des Behandlungsstroms $I_{HF}$. Wenn die Frequenz des Behandlungsstroms $I_{HF}$ 350 kHz beträgt, so eignet sich für den Messstromgenerator eine Frequenz von (etwa) 70 kHz. Der Messstromgenerator 32 kann von einer Auswerteinrichtung 30 über eine entsprechende Steuerleitung gesteuert werden.

Parallel zum Messstromgenerator 32 ist eine Spannungsmesseinrichtung 35 vorgesehen, welcher ein Filter 34 vorgeschaltet ist. Das Filter 34 ist in seiner Durchlassfrequenz auf die Frequenz des Messstroms $I_M$ abgestimmt, so dass die Spannungsmesseinrichtung 35 lediglich die Amplitude der Spannungsanteile wiedergibt, welche durch den Abfall des Messstroms $I_M$ über der Messstrecke zwischen der Hauptelektrode 10 und der Messelektrode 14 bei Fließen des Messstroms $I_M$ entsteht. Ein entsprechendes Messspannungssignal wird der Auswerteinrichtung 30 zugeführt.

Weiterhin enthält die Impedanz-Messeinrichtung 20 eine zweite Spannungsmesseinrichtung mit einem vorgeschalteten Filter 33, dessen Durchlassfrequenz auf die Frequenz (350 kHz) des Behandlungsstroms $I_{HF}$ abgestimmt ist. Das Ausgangssignal der zweiten Spannungsmesseinrichtung, welches der Auswerteinrichtung 30 zugeführt wird, gibt also den Spannungsabfall wieder, der den zwischen der Messelektrode 14 und der Hauptelektrode 10 fließenden Behandlungsstrom entsteht.

Die Auswerteinrichtung 30 ist mit einer Anzeige bzw. Signalerzeugungseinrichtung 31 verbunden.

Im Betrieb wird nun nach dem Aufkleben der Elektrodeneinrichtung auf einen Hautabschnitt 5 eines Patienten kontinuierlich oder intermittierend durch den Generator 32 ein Messstrom IM erzeugt. Die durch die Spannungsmesseinrichtung 35 gemessene Spannung gibt somit (bei konstantem Messstrom $I_M$) ein Maß für die Impedanz wieder, die zwischen der Messelektrode 14 und der Hauptelektrode 10 vorliegt. Steigt die Impedanz gegenüber einem vorgebbaren (bzw. aus früheren Untersuchungen festgestellten) "Normalwert" an, so kann daraus geschlossen werden, dass der Widerstand zwischen der Messelektrode 14 und dem Gewebe 6 und/oder der Widerstand zwischen der Hauptelektrode 10 und dem Gewebe 6 sehr hoch ist bzw. angestiegen ist. Daran wiederum kann erkannt werden, dass ein Kontaktfehler vorliegt, die Elektrode sich also abgelöst hat oder aus anderen Gründen ein zu hoher Widerstand zwischen der Hauptelektrode 10 und/oder der Messelektrode 14 und dem Hautabschnitt 5 bzw. dem darunter liegenden Gewebe 6 besteht. Dementsprechend kann über die Auswerteinrichtung 30 eine entsprechende Anzeige auf der Anzeigeeinrichtung bzw. Signalerzeugungseinrichtung 31 abgegeben werden, um das Operationspersonal davor zu warnen, dass nun die Gefahr einer zu hohen Stromdichte und damit übergroßen Erwärmung des Gewebes bzw. Hautabschnittes 5 vorliegt.

Wenn weiterhin über die zweite Spannungsmesseinrichtung 36 bei Fließen eines Behandlungsstroms $I_{HF}$ ein Spannungsabfall festgestellt wird, der eine vorbestimmte Schwelle überschreitet, so kann hieraus wiederum geschlossen werden, dass unkorrekte Widerstandsverhältnisse vorliegen. Auch hieraus kann auf eine zu hohe Stromdichte und darum Gefahr einer übergroßen Erwärmung geschlossen werden.

Mit der soeben dargestellten Ausführungsform der Erfindung kann - wie dies in Fig. 11 gezeigt ist - auch eine weitere Problematik festgestellt werden. Dann nämlich, wenn nahe unter der Elektrode 10 bzw. dem dazu gehörigen Hautabschnitt 5 ein Gefäß 7 verläuft, das Gewebe 6 zusätzlich möglicherweise auch relativ viel Fett umfasst, ergibt sich aufgrund der nun vorliegenden inhomogenen Widerstandsverhältnisse ein erhöhter Stromfluss zwischen dem Bereich des Gefäßes 7 und der Hauptelektrode 10. In diesem Fall findet also ein verringerter Stromfluss zwischen der als elektrisch leitender Ring wirkenden Messelektrode 14 und der Hauptelektrode 10 statt, was durch die zweite Spannungsmesseinrichtung 36 festgestellt und der Auswerteinrichtung 30 "mitgeteilt" wird. In diesem Fall kann über die Anzeigeeinrichtung 31 ein Signal abgegeben werden, welches dem Operationspersonal mitteilt, dass aufgrund der vorliegenden Widerstandsverhältnisse mit einer gebietsweise erhöhten Stromdichte zu rechnen ist, die bekanntlich zu einer übergroßen Erwärmung des Gewebes 6 bzw. der unter der Hauptelektrode 10 liegenden Haut führen kann.

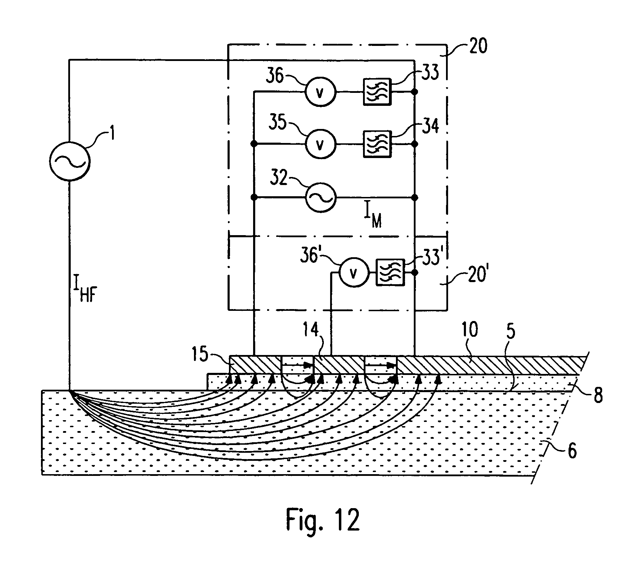

Bei der in Fig. 12 gezeigten Ausführungsform sind mehrere als elektrisch leitende Ringe ausgebildete Messelektroden 14, 15 vorgesehen. Weiterhin sind auch mehrere zweite Spannungsmesseinrichtungen 36, 36' vorgesehen, so dass ein Verlauf des Spannungsabfalls bei Fließen eines Behandlungsstroms $I_{HF}$ zwischen der Hauptelektrode 10 und der ersten Messelektrode 14 und der zweiten Messelektrode 15 feststellbar ist. Anhand des Verlaufes des Spannungsabfalls kann besser als bei der Ausführungsform nach Fig. 11 festgestellt werden, ob Inhomogenitäten im Gewebe 6 bzw. im gesamten Strompfad zwischen dem Generator 1 und der Hauptelektrode 10 vorliegen. Weiterhin kann auch das randseitige Ablösen der Elektrodeneinrichtung noch besser, genauer und in seiner Bedeutsamkeit leichter einschätzbar festgestellt werden. Wenn beispielsweise der Spannungsabfall zwischen der Messelektrode 14 und 15 zwar ein Ablösen signalisiert, zwischen der Hauptelektrode 10 und der Messelektrode 14 jedoch noch ein korrekter Wert vorliegt, so kann daraus geschlossen werden, dass noch keine "Gefahr" für den Patienten besteht, wohl aber der korrekte Sitz der Neutralelektrode nunmehr überprüft werden soll.

Folgende Parameter können durch die Auswertschaltung bestimmt werden:

$I_M$     Messstrom zur Messung des Übergangswiderstandes $R_{UEB}$

UM, Ring     Spannungsabfall durch $I_M$ zwischen elektrisch leitendem Ring und Innenfläche, wird zur Berechnung des Übergangswiderstandes in Zusammenhang mit $I_M$ herangezogen

$U_{HF}$, Ring     HF-Spannung (350 kHz) zwischen Hauptelektrode und elektrisch leitendem Ring. Zusammen mit dem Übergangswiderstand ein Maß für den Strom, der "über" den elektrisch leitenden Ring fließt

$I_{HF}$     Gesamtstrom, der über die Neutralelektrode zum Generator fließt

$$R_{UEB} = \frac{U_{M,Ring}}{I_M}$$

$$P_{HF,innen} = (I_{HF} - I_{HF,Ring})^2 * \left( R_{UEB} * \frac{I_{HF,Ring}}{I_{HF} - I_{HF,Ring}} \right) = R_V * (I_{HF} - I_{HF,Ring})^2$$

$$\bar{R}_V = \frac{I_{HF,Ring}}{I_{HF} - I_{HF,Ring}} * \bar{R}_{UEB}$$

$$P_{HF,\,Ring} = U_{HF,\,Ring} * I_{HF,\,Ring} = \frac{U^2_{HF,Ring}}{R_{UEB}}$$

$R_{UEB}$     Übergangswiderstand zur Bestimmung der Anlagequalität und der Berechnung über den Ring verteilten Stroms

$I_{HF,\,Ring}$     HF-Strom, der auf den elektrisch leitenden Ring fließt und verteilt wird

$P_{HF,\,innen}$     Leistung, die am Rand der Hauptelektrode in Wärme umgesetzt wird

$P_{HF,\,Ring}$     Leistung, die am elektrisch leitenden Ring in Wärme umgesetzt wird

$R_v$     Virtueller Widerstand des Gewebes unter der Neutralelektrode, über den der Teil des HF-Stroms fließt, der nicht über den Ring verteilt wird. Dient als Hilfsgröße zur Leistungsberechnung

Aus den beiden berechneten Leistungen $P_{HF, innen}$ und $P_{HF, Ring}$ sind Abschätzungen der Temperaturerhöhung an den Rändern der Neutralelektrode möglich, da diese Leistungen in einem geometrisch näherungsweise bekannten Gebiet umgesetzt werden. Hierzu ist eine allgemeine Annahme der thermischen Leitfähigkeit des darunter liegenden Gewebes möglich, da zum einen das betroffene Gebiet von den Ausmaßen her sehr eingeschränkt ist und zum anderen unter der Elektrode keine direkten Luftströmungen oder sonstige externe Einflüsse auftreten können.

Aus Obigem geht hervor, dass folgende Vorteile von besonderer Bedeutung sind:

Durch die Erfindung wird eine bessere Erkennung einer Ablösung der Elektrodeneinrichtung am Rand ermöglicht. An jeder Kante der "Neutralelektrode" wird eine Ablösung von der Haut mit derselben Genauigkeit erkannt. Da in dem Randbereich auch die größte Stromdichte auftritt, ist hier eine exakte Überwachung nötig. Demgegenüber ist bei den bisherigen Elektrodenformen die Erkennung dieser wichtigen Parameter senkrecht zur Gelbrücke ungenauer als parallel dazu. Insbesondere ist dieser Nachteil bei den handelsüblichen, zweigeteilten Neutralelektroden (siehe z.B. EP 0 390 937 B1) bedeutsam.

Durch Messung der HF-Spannung zwischen dem elektrisch leitenden Ring und der Hauptelektrode kann durch den bekannten Übergangswiderstand der Strom ermittelt werden, der vom elektrisch leitenden Ring verteilt wird. Somit kann eine Aussage über das Verhältnis des Gesamtstroms zum Strom über den elektrisch leitenden Ring getroffen und dadurch bestimmt werden, wie effektiv die Verteilungswirkung des elektrisch leitenden Ringes ist.

Durch messtechnische Ermittlungen der Stromverteilung zwischen dem unter dem Ring liegenden Gewebe und dem elektrisch leitenden Ring können auch Rückschlüsse auf die Widerstandsverhältnisse der tieferen, stromführenden Gewebeschichten gezogen werden.

Wenn sich unter der den Behandlungsstrom $I_{HF}$ ableitenden Fläche der Neutralelektrode ein sehr gut leitfähiges Gewebe mit einem geringen Flächenanteil befindet, z.B. ein großes Blutgefäß nahe unter der Hautoberfläche (womöglich umgeben von Fettgewebe), fließt darüber ein großer Teil des Stroms, was aufgrund der kleinen Fläche zu hohen Stromdichten und damit Verbrennungen an diesen Stellen führen kann. Solche Fälle sind aus der Vergangenheit bekannt. Dieses Problem kann bei der beschriebenen Anordnung bzw. dem beschriebenen Verfahren dadurch erkannt werden, dass nur noch ein sehr geringer Anteil des Behandlungsstroms $I_{HF}$ über den elektrisch leitenden Ring fließt. Folglich muss der restliche Behandlungsstrom auf einem Weg mit geringerer Leitfähigkeit direkt unter die Hauptelektrode gelangen können.

Mit den bekannten Strom- und Widerstandsverhältnissen kann die zugehörige Verlustleistung berechnet werden. Somit können Aussagen über eine mögliche Temperaturerhöhung in den Randbereichen der Elektrode getroffen werden, wo die Temperaturerhöhung auch am größten ist. Das exakte thermische Verhalten des Gewebes ist zwar von Fall zu Fall verschieden, da es sich aber um ein geometrisch sehr eingegrenztes Gebiet unter der Elektrode handelt, kann mit hoher Wahrscheinlichkeit eine unzulässige Temperaturerhöhung mit guter Genauigkelt abgeschätzt werden.

Die Orientierung der erfindungsgemäßen Elektrode zum Operationsfeld ist nahezu bedeutungslos (von der Anschlussfahne abgesehen). Prinzip bedingt ist das Auftreten eines unsymmetrischen Behandlungsstroms $I_{HF}$ nicht mehr möglich, da es nur eine einzige den Behandlungsstrom leitende Fläche (die Hauptelektrode 10) gibt. Damit werden mögliche Fehler durch eine nicht richtig zum Operationsfeld ausgerichtete Neutralelektrode vermieden.

Bezugszeichenliste

| | |
|---|---|
| 1 | HF-Generator |
| 2 | Elektrochirurgisches Instrument |
| 3 | Neutralelektrode |
| 5 | Hautabschnitt |
| 6 | Gewebe/Körper |
| 7 | Gefäß |
| 8 | Leitgel |
| 9 | Trägerfolie |
| 10 | Hauptelektrode |
| 11 | elektrisch leitender Ring |
| 12 | Spalt |
| 13 | Anschlussfahne |
| 14 | Messelektrode |
| 15 | Messelektrode |
| 16 | Messelektrode |

| 17 | Messelektrode |
| 20 | Impedanz-Messeinrichtung |
| 30 | Auswerteinrichtung |
| 31 | Anzeige/Signal-Erzeugungseinrichtung |
| 32 | Messstromgenerator |
| 33, 33' | Filter($I_{HF}$) |
| 34 | Filter ($I_M$) |
| 35 | Spannungsmesseinrichtung ($U_M$) |
| 36, 36' | Spannungsmesseinrichtung ($U_{HF}$) |

**Patentansprüche**

1. Elektrodeneinrichtung, insbesondere Neutralelektrode, zum Einleiten eines hochfrequenten Behandlungsstroms ($I_{HF}$) über einen Hautabschnitt (5) in einen menschlichen oder tierischen Körper (6), insbesondere bei einer monopolaren elektrochirurgischen Behandlung, umfassend

   - einen Behandlungsstromgenerator zur Erzeugung des hachfrequenten Behandlungsstroms,
   - mindestens eine Hauptelektrode (10) zum Einleiten des Behandlungsstroms ($I_{HF}$) in den Körper (6),
   - eine Impedanz-Messeinrichtung (20) zum Messen einer Impedanz zwischen der Hauptelektrode (10) und dem Körper (6) und zum Erzeugen eines Impedanzsignals,
   - eine mit der Impedanz-Messeinrichtung (20) verbundene Auswerteinrichtung (30) zur Erzeugung eines Auswertsignals betreffend die Impedanz und/oder eine zumindest mögliche Erwärmung des Körpers (6) und/oder des Hautabschnittes (5), wobei
   - mindestens eine zur Hauptelektrode (10) beabstandet angeordnete Messelektrode (14 - 17) vorgesehen ist und die Impedanz-Messeinrichtung (20) mindestens einen mit der Hauptelektrode (10) und der Messelektrode (14 - 17) verbundenen Messstromgenerator (32) zur Erzeugung eines hochfrequenten Messstroms (IM) umfasst, der zwischen der Messelektrode (14 - 17) und der Hauptelektrode (10) fließt, und wobei
   - der Messstrom (IM) eine vom Behandlungsstrom ($I_{HF}$) derart verschiedene Frequenz oder Phasenlage aufweist, dass der Messstrom ($I_M$) vom Behandlungsstrom ($I_{HF}$) durch Filtereinrichtungen (33, 34) trennbar ist und die Frequenz des Messstroms ($I_M$) niedriger ist als die Frequenz des Behandlungsstroms ($I_{HF}$),
   - die Impedanz-Messeinrichtung (20) eine Spannungsmesseinrichtung (35) zur Messung eines Spannungsabfalls ($U_M$) zwischen der Hauptelektrode (10) und der Messelektrode (14 - 17), der durch Durchleiten des Messstroms ($I_M$) durch die Messstrecke zwischen der Hauptelektrode und der Messelektrode entsteht, umfasst

   **dadurch gekennzeichnet, dass**

   - die Impedanz-Messeinrichtung (20) eine zweite Spannungsmesseinrichtung (36, 36') zur Messung eines Spannungsabfalles ($U_{HF}$) zwischen der Hauptelektrode (10) und der Messelektrode (14 - 17) der durch Fließen des Behandlungsstromes zwischen der Hauptelektrode und der Messelektrode entsbeht, ($I_{HF}$) umfasst, wobei die zweite Spannungsmesseinrichtung einen vorgeschalteten Filter aufweist, dessen Durchlassfrequenz auf die Frequenz des Behandlungsstroms abgestimmt ist.

2. Elektrodeneinrichtung nach einem der vorhergehenden Ansprüche, **dadurch gekennzeichnet, dass** ein Rand der Messelektrode (14 - 17) in equidistantem Abstand um einen Rand der Hauptelektrode (10) angeordnet ist.

3. Elektrodeneinrichtung nach einem der vorhergehenden Ansprüche, **dadurch gekennzeichnet, dass** die Messelektrode (14 - 17) als elektrisch leitender Ring ausgebildet ist.

4. Elektrodeneinrichtung nach einem der vorhergehenden Ansprüche, **dadurch gekennzeichnet, dass** eine Vielzahl von jeweils elektrisch leitende Ringe bildenden Messelektroden (14 - 17) und eine Vielzahl von Impedanz-Messeinrichtungen (20, 20') vorgesehen und die Auswerteinrichtung (30) derart ausgebildet ist, dass ein Verlauf der Spannungsabfälle ($U_{HF}$) des Behandlungsstromes ($I_{HF}$) von der Hauptelektrode (10) zu den Messelektroden (14 - 17) feststellbar ist.

5. Elektrodeneinrichtung nach einem der vorhergehenden Ansprüche, **dadurch gekennzeichnet, dass** die Auswerteinrichtung (30) derart ausgebildet ist, dass ein Warnsignal dann abgegeben wird, wenn ein durch den Behandlungsstrom ($I_{HF}$) bewirkter Spannungsabfall ($U_{HF}$) zur Messelektrode (14 - 17) eine vorbestimmte Schwelle überschreitet.

**Claims**

**1.** Electrode device, in particular neutral electrode, for introducing a high-frequency treatment current ($I_{HF}$) into a human or animal body (6) via a section (5) of skin, in particular as part of a monopolar electrosurgical treatment, comprising

- a treatment-current generator for generating the high-frequency treatment current,
- at least one main electrode (10) for introducing the treatment current ($I_{HF}$) into the body (6),
- an impedance-measuring device (20) for measuring an impedance between the main electrode (10) and the body (6) and for generating an impedance signal,
- an evaluation device (30), which is connected to the impedance-measuring device (20), for generating an evaluation signal relating to the impedance and/or at least possible heating of the body (6) and/or of the section (5) of skin, with
- at least one measurement electrode (14-17), which is arranged with a spacing from the main electrode (10), being provided, and the impedance-measuring device (20) comprising at least one measurement-current generator (32), which is connected to the main electrode (10) and the measurement electrode (14-17), for generating a high-frequency measurement current ($I_M$) which flows between the measurement electrode (14-17) and the main electrode (10), and with
- the measurement current ($I_M$) having a frequency or phase angle which differs from the treatment current ($I_{HF}$) in such a way that the measurement current ($I_M$) can be separated from the treatment current ($I_{HF}$) by filter devices (33, 34) and the frequency of the measurement current ($I_M$) is lower than the frequency of the treatment current ($I_{HF}$),
- the impedance-measuring device (20) comprising a first voltage-measuring device (35) for measuring a voltage drop ($U_M$) between the main electrode (10) and the measurement electrode (14-17), this voltage drop being the result of the measurement current ($I_M$) being passed through the measurement section between the main electrode and the measurement electrode,

**characterized in that**

- the impedance-measuring device (20) comprises a second voltage-measuring device (36, 36') for measuring a voltage drop ($U_{HF}$) between the main electrode (10) and the measurement electrode (14-17), this voltage drop being the result of the treatment current ($I_{HF}$) flowing between the main electrode and the measurement electrode, with the second voltage-measuring device having an upstream filter, the pass frequency of this filter being matched to the frequency of the treatment current.

**2.** Electrode device according to either of the preceding claims, **characterized in that** an edge of the measurement electrode (14-17) is arranged with an equidistant spacing around an edge of the main electrode (10).

**3.** Electrode device according to either of the preceding claims, **characterized in that** the measurement electrode (14-17) is in the form of an electrically conductive ring.

**4.** Electrode device according to one of the preceding claims, **characterized in that** a large number of measurement electrodes (14-17), which in each case form electrically conductive rings, and a large number of impedance-measuring devices (20, 20') are provided, and the evaluation device (30) is formed in such a way that a profile of the voltage drops ($U_{HF}$) of the treatment current ($I_{HF}$) from the main electrode (10) to the measurement electrodes (14-17) can be determined.

**5.** Electrode device according to one of the preceding claims, **characterized in that** the evaluation device (30) is formed in such a way that a warning signal is output when a voltage drop ($U_{HF}$) to the measurement electrode (14-17), this voltage drop being caused by the treatment current ($I_{HF}$), exceeds a predetermined threshold.

**Revendications**

**1.** Système d'électrodes, notamment d'électrodes neutres, destiné à introduire un courant de traitement à haute fréquence ($I_{HF}$) par l'intermédiaire d'une section de peau (5) dans un organisme humain ou animal (6), notamment lors d'un traitement électro-chirurgical monopolaire, comprenant :

- un générateur de courant de traitement destiné à générer le courant de traitement à haute fréquence,
- au moins une électrode principale (10) destinée à introduire le courant de traitement ($I_{HF}$) dans l'organisme (6),
- un dispositif de mesure d'impédance (20) destiné à mesurer une impédance entre l'électrode principale (10) et l'organisme (6) et à générer un signal d'impédance,
- un dispositif d'analyse (30) connecté au dispositif de mesure d'impédance (20) pour générer un signal d'analyse concernant l'impédance et/ou un chauffage au moins possible de l'organisme (6) et/ou de la section de peau (5), dans lequel
- il est prévu au moins une électrode de mesure (14-17) espacée de l'électrode principale (10) et le dispositif de mesure d'impédance (20) comprend au moins un générateur de courant de mesure (32) connecté à l'électrode principale (10) et à l'électrode de mesure (14-17) pour générer un courant de mesure à haute fréquence ($I_M$) passant entre l'électrode de mesure (14-17) et l'électrode principale (10), et dans lequel
- le courant de mesure ($I_M$) présente une fréquence ou une position de phase qui diffère du courant de traitement ($I_{HF}$) de telle manière que le courant de mesure ($I_M$) puisse être séparé du courant de traitement ($I_{HF}$) par des dispositifs de filtrage (33, 34) et que la fréquence du courant de mesure ($I_M$) soit plus faible que la fréquence du courant de traitement ($I_{HF}$),
- le dispositif de mesure d'impédance (20) comprend un premier dispositif de mesure de tension (35) destiné à mesurer, entre l'électrode principale (10) et l'électrode de mesure (14-17), une chute de tension ($U_M$) qui est provoquée par le passage du courant de mesure ($I_M$) à travers l'espace de mesure entre l'électrode principale et l'électrode de mesure,

**caractérisé en ce que**

- le dispositif de mesure d'impédance (20) comprend un second dispositif de mesure de tension (36, 36') destiné à mesurer, entre l'électrode principale (10) et l'électrode de mesure (14-17), une chute de tension ($U_{HF}$) qui est provoquée par le passage du courant de traitement ($I_{HF}$) entre l'électrode principale et l'électrode de mesure, dans lequel le second dispositif de mesure de tension présente un filtre connecté en amont dont la fréquence passante est accordée à la fréquence du courant de traitement.

2. Système d'électrodes selon l'une quelconque des revendications précédentes, **caractérisé en ce qu'**un bord de l'électrode de mesure (14-17) est disposé à un intervalle équidistant sur le pourtour d'un bord de l'électrode principale (10).

3. Système d'électrodes selon l'une quelconque des revendications précédentes, **caractérisé en ce que** l'électrode de mesure (14-17) est réalisée sous la forme d'un anneau électriquement conducteur.

4. Système d'électrodes selon l'une quelconque des revendications précédentes, **caractérisé en ce qu'**il est prévu une pluralité d'électrodes de mesure (14-17) formant des anneaux électriquement conducteurs respectifs et une pluralité de dispositifs de mesure d'impédance (20, 20') et **en ce que** le dispositif d'analyse (30) est conçu de manière à pouvoir déterminer une évolution de la chute de tension ($U_{HF}$) du courant de traitement ($I_{HF}$) de l'électrode principale (10) aux électrodes de mesure (14-17).

5. Système d'électrodes selon l'une quelconque des revendications précédentes, **caractérisé en ce que** le dispositif d'analyse (30) est conçu de manière à ce qu'un signal d'alarme soit délivré à l'instant où une chute de tension ($U_{HF}$) provoquée par le courant de traitement ($I_{HF}$) passant vers l'électrode de mesure (14-17) dépasse un seuil prédéterminé.

EP 2 043 539 B1

Fig. 1
Stand der Technik

Fig. 2
Stand der Technik

Fig. 3

Fig. 4

Fig. 5

Fig. 6

10

Fig. 7

Fig. 8

Fig. 9

Fig. 10

Fig. 11

Fig. 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1173095 B1
- EP 0390937 B1
- EP 1051949 B1
- DE 3239640 A